# EUROPEAN PATENT SPECIFICATION

(11) **EP 2 561 098 B1**
(45) Date of publication and mention of the grant of the patent: **09.09.2015**
(21) Application number: 11771401.4
(22) Date of filing: 21.04.2011
(51) Int. Cl.: C12Q 1/68, G01N 33/542

(54) **TETHERED ENZYME MEDIATED NUCLEIC ACID DETECTION**
DURCH VERANKERTE ENZYME VERMITTELTE NUKLEINSÄUREERKENNUNG
DÉTECTION D'ACIDES NUCLÉIQUES À MÉDIATION PAR UNE ENZYME CAPTIVE

(30) Priority: 23.04.2010 AU 2010901732
(43) Date of publication of application: 27.02.2013
(73) Proprietor: Australian Centre for Plant Functional Genomics Pty Ltd., Urrbrae, South Australia 5064 (AU)
(72) Inventor: FLETCHER, Stephen, John, Toowong Queensland 4066 (AU)
(74) Representative: Banford, Paul Clifford
(86) International application number: PCT/AU2011/000453
(87) International publication number: WO 2011/130789

(56) References cited:
- WO-A1-2008/122088
- WO-A2-2009/017861
- US-A- 5 118 605
- US-A- 6 114 117
- US-A1- 2010 041 049
- BEYER S ET AL: "A modular DNA signal translator for the controlled release of a protein by an aptamer", NUCLEIC ACIDS RESEARCH, OXFORD UNIVERSITY PRESS, SURREY, GB, vol. 34, no. 5, 17 March 2006 (2006-03-17) , pages 1581-1587, XP008119760, ISSN: 0305-1048, DOI: 10.1093/NAR/GKL075 [retrieved on 2006-03-17]
- FLETCHER, S.J. ET AL.: 'Toward specific detection of Dengue virus serotypes using a novel modular biosensor' BIOSENSORS AND BIOELECTRONICS vol. 26, no. 4, 2010, pages 1696 - 1700, XP027546904

## Description

### FIELD OF THE INVENTION

The present invention relates to methods useful for the detection of a target nucleotide sequence in a sample. In general, the methods of the present invention are predicated on the use of a tethered reporter enzyme.

### BACKGROUND OF THE INVENTION

A range of molecular technologies for the detection of a target analyte in a sample have been developed. Such methods have application in, for example, public health, the detection of pathogens in food or water, epidemiological studies, genetically modified organism (GMO) detection, medicine, clinical diagnosis, disease susceptibility diagnosis, tissue typing, blood screening, forensic medicine, bioweapon detection, molecular toxicology, gene therapy, and DNA tagging, among many other applications.

Current methods for detecting an analyte such as a nucleic acid generally involve one, or a combination of, molecular techniques. These techniques generally fall into three groups loosely defined as sequence-specific detection, sequence-specific enrichment and signal amplification.

Most detection techniques gain their sequence specificity through base pairing of complementary probes or oligonucleotides to a sequence of interest within the target nucleic acid sample.

The most commonly used nucleic acid detection methods, namely, polymerase chain reaction (PCR) and Southern or Northern blotting, differ in how they proceed from this point. The PCR method enriches a target DNA through a series of amplification cycles and signal detection can be, for example, through the use of stains, fluorescence or radiolabeling. Southern or Northern blotting involve no nucleic acid (DNA or RNA, respectively) enrichment step, but use high-energy radioactive isotopes (e.g. ²³P) for signal amplification. These extensively used techniques, though highly developed, still retain significant drawbacks. For PCR, the equipment required is expensive, the process is time-consuming and the degree of expertise required is high. Southern blotting often uses hazardous radioactive labeling, takes up to a week to complete, and requires large amounts of substrate DNA.

A biosensor for detecting analytes such as nucleic acids which is target-specific and sensitive would be desirable. Furthermore, such a technique would also ideally require a low capital input (particularly in the case of equipment requirement), minimal expertise or technique-specific training, and provide quick and accurate results.

Reference to any prior art in this specification is not, and should not be taken as, an acknowledgment or any form of suggestion that this prior art forms part of the common general knowledge in any country.

### SUMMARY OF THE INVENTION

The present invention is predicated in part on the development of biosensors and methods of use thereof for detecting analytes in biological samples.

Accordingly, the present invention provides a method for detecting a target nucleotide sequence in a sample, the method comprising:
providing a reporter enzyme tethered to a solid support via a tether nucleotide sequence;
providing a linker nucleotide sequence comprising a binder which can hybridise to the target nucleotide sequence, and a trigger which can hybridise to the tether nucleotide sequence only when the binder nucleotide sequence is bound to the target;
providing a primary nuclease which digests at least the tether nucleotide sequence in a hybrid formed between the trigger and the tether nucleotide sequence;
contacting a sample comprising the target nucleotide sequence with the reporter enzyme tethered to a solid support, the linker nucleotide sequence, and the nuclease, under suitable conditions wherein the binder hybridises to the target nucleotide sequence, the trigger then hybridises to the tether nucleotide sequence, the nuclease digests at least the tether nucleotide sequence in the hybrid formed between the trigger and the tether nucleotide sequence, and the reporter enzyme is released from the solid support; and
detecting the activity of the reporter enzyme released from the solid support wherein the activity of the reporter enzyme released from the solid support Is indicative of the presence of the target in the sample.

### BRIEF DESCRIPTION OF THE FIGURES

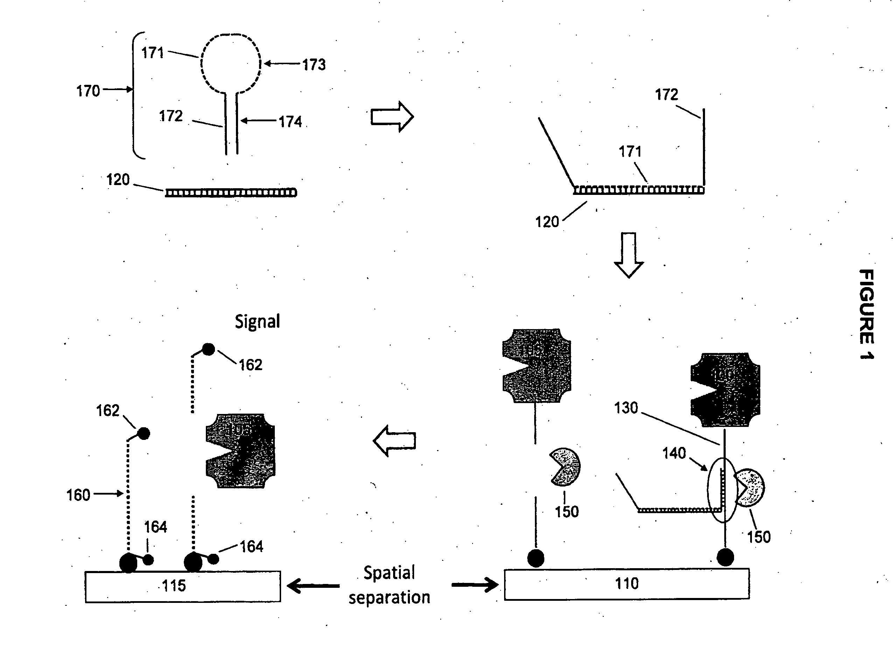

Figure 1 is a schematic representation showing target-mediated separation of a tethered reporter enzyme and subsequent signal generation. Following hybridisation of a linker to a target nucleic acid, the trigger sequence in the stem of the linker may bind to a tether nucleotide sequence tethering a reporter enzyme to a solid support. A primary nuclease is then able to cleave the tether, allowing the previously tethered enzyme to be free in solution and thus act on a spatially-separated signaling substrate.

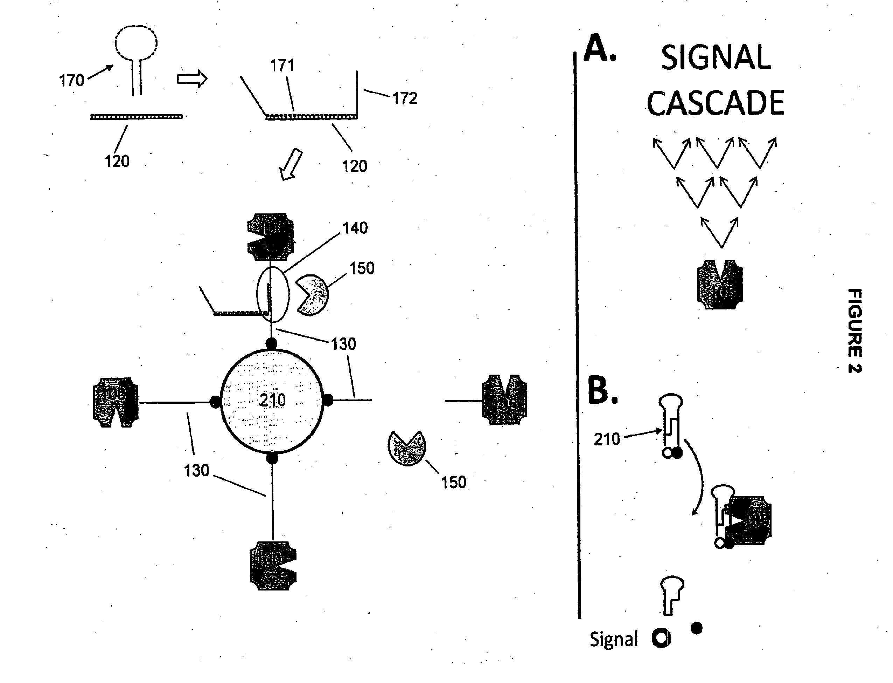

Figure 2 is a schematic representation showing temporal separation of a tethered reporter enzyme and its signaling substrate through the use of magnetic beads. A reporter enzyme may be tethered to magnetic beads. Cleavage of the tether due to the presence of the target nucleic acid allows the previously tethered reporter enzyme to be free in solution. Following magnetic removal of the beads, the free enzyme is able to act on subsequently-added signaling substrate. Panel A. The free reporter enzyme (e.g. thrombin) may generate a signal cascade. Panel B. The free reporter enzyme may be a restriction endonuclease and cleave multiple signaling molecules containing the appropriate recognition sequence.

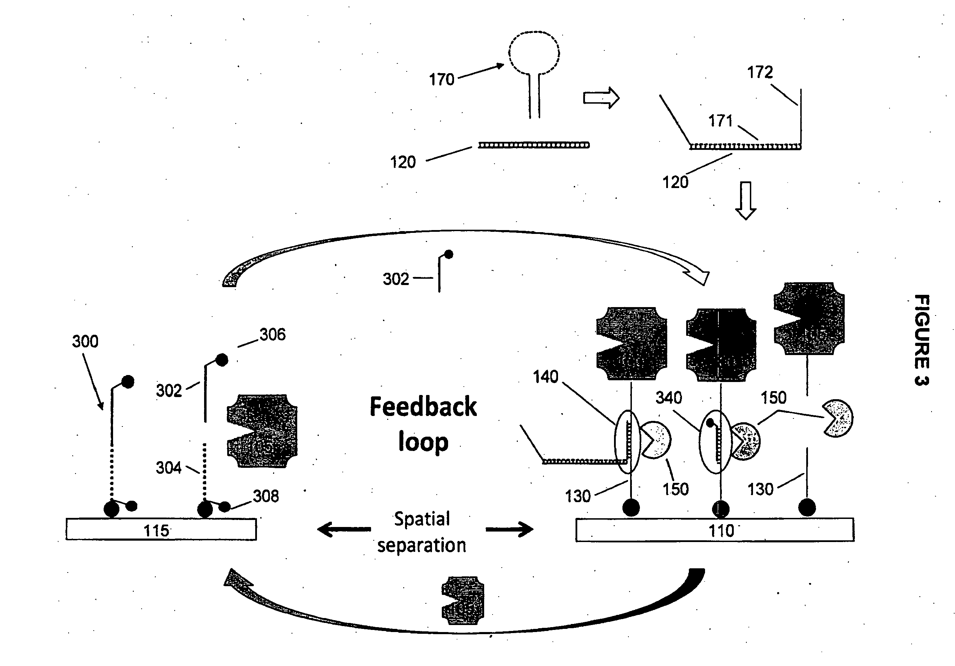

Figure 3 is a schematic representation showing an incorporated feedback loop allowing the separation of multiple tethered reporter enzyme molecules from their solid support for each target nucleic acid present in solution. Reporter enzyme molecules, free in solution due to the presence of the target nucleic acid, can cleave a portion of the spatially-separated intermediary molecule. A feedback trigger released by digestion of the intermediary molecule can create the substrate for the primary nuclease by hybridising to the tether between an additional reporter enzyme molecule and the solid support. Through the action of the primary nuclease, the additional reporter enzyme molecule becomes free in solution and may act on a further intermediary molecule to release a further feedback trigger. In this example, the activity of the reporter enzyme may be detected by signal generated as a result of the digestion of the intermediary molecule.

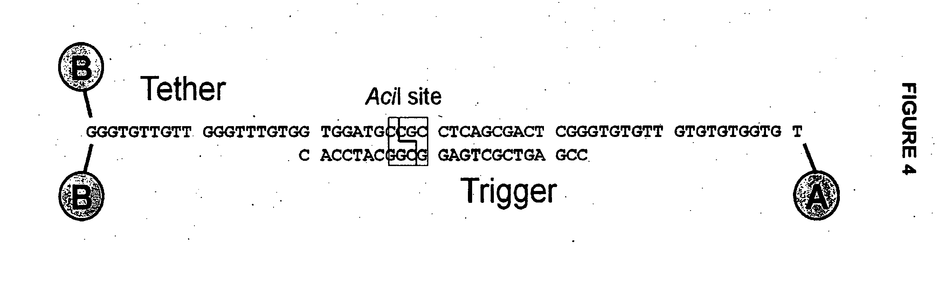

Figure 4 is a graphical representation of an embodiment of a tether/trigger duplex. A recognition and cleavage site for the restriction endonuclease *Aci*I is shown in the grey-shaded box. The 5' terminus of the tether is labeled with two biotin molecules (B) and the 3' terminus labeled with an amine group (A). The biotin labels allow for a strong bond to form with a streptavidin-coated surface while the amine label can be used for covalent conjugation of the tether to the reporter enzyme.

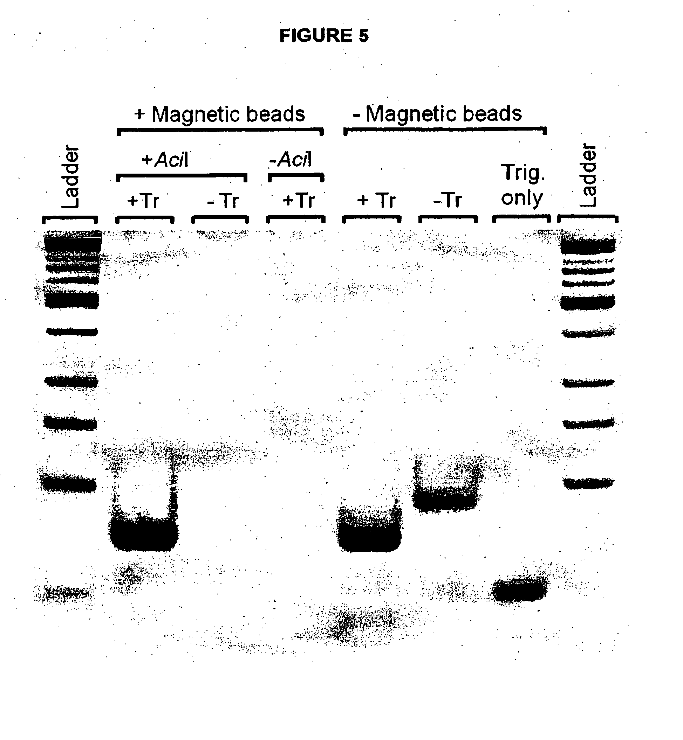

Figure 5 is a pictorial representation of a gel-shift assay showing the specific digestion of a tether in the presence of a trigger. A DNA band of the correct size was evident for the treatment to which the trigger had been added (+Tr) and absent in the no trigger (-Tr) treatment. This indicated the trigger had bound to the tether and the duplex was specifically cleaved by *Aci*I, with the separated non-bead-bound portion remaining after bead removal. The lack of bands in the magnetic bead control treatments indicated that, as desired, in the absence of the trigger or the absence of *Aci*I, the tether remained intact and was removed with the beads. For the non-bead-bound control treatments (- magnetic beads), the presence of the trigger allowed *Aci*I to cleave the duplex, leading to a band of reduced size in comparison to non-cleaved no trigger control.

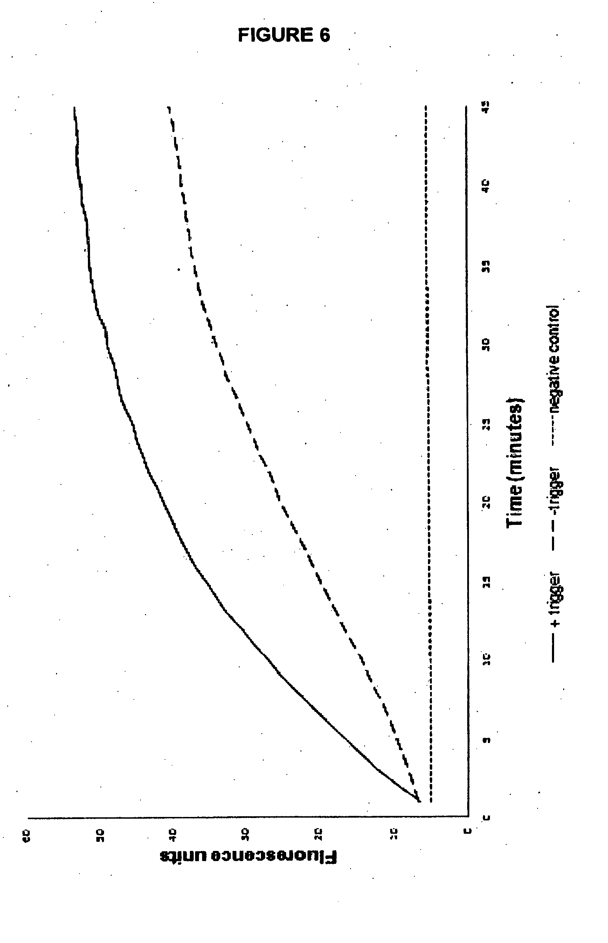

Figure 6 is a graphical representation showing the addition of the trigger results in an increase in the fluorescent signal over time. The trigger is able to hybridise to the bead-bound tether, with *Aci*I subsequently cleaving the duplex and releasing RNase A from the magnetic bead. Upon removal of the magnetic beads and addition of the signaling molecule, the remaining RNase A can produce a detectable signal. The presence of a reduced signal in the -trigger control indicates that some non-conjugated RNase A remains in the reaction.

### DESCRIPTION OF THE INVENTION

In a first aspect, the present invention provides a method for detecting a target nucleotide sequence in a sample, the method comprising:
providing a reporter enzyme tethered to a solid support via a tether nucleotide sequence;
providing a linker nucleotide sequence comprising a binder which can hybridise to the target nucleotide sequence, and a trigger which can hybridise to the tether nucleotide sequence only when the binder nucleotide sequence is bound to the target;
providing a primary nuclease which digests at least the tether nucleotide sequence in a hybrid formed between the trigger and the tether nucleotide sequence;
contacting a sample comprising the target nucleotide sequence with the reporter enzyme tethered to a solid support, the linker nucleotide sequence, and the nuclease, under suitable conditions wherein the binder hybridises to the target nucleotide sequence, the trigger then hybridises to the tether nucleotide sequence, the nuclease digests at least the tether nucleotide sequence in the hybrid formed between the trigger and the tether nucleotide sequence, and the reporter enzyme is released from the solid support; and
detecting the activity of the reporter enzyme released from the solid support wherein the activity of the reporter enzyme released from the solid support is indicative of the presence of the target in the sample.

The "sample" in which a target may be detected may be any sample that putatively contains the target. For example, the sample may be a biological sample including samples derived from an organism, a sample derived from an organ, a sample containing one or more cells, a blood sample, a plasma sample, a CSF fluid sample, an amniotic fluid sample and the like; an environmental sample such as a water, air or soil sample; a food or beverage sample; and the like. The samples contemplated herein may be used in a crude form, or the samples may be processed for use in accordance with the present invention. For example, the sample may have one or more extraction or purification steps performed thereon in order to purify or semi-purify the target present in the sample.

As set out above, the present invention uses a "linker nucleotide sequence" which comprises a binder which can hybridise to the target nucleotide sequence, and a trigger which can hybridise to the tether nucleotide sequence when the binder nucleotide sequence is bound to the target.

The use of a linker nucleotide sequence provides a significant advantage in that it allows the method of the present invention to be modular. That is, binder sequences for different targets can be incorporated into a linker, while the trigger sequence may be optimised to hybridise to a particular tether nucleotide sequence. This provides an advantage in that an assay based on a single trigger-tether hybridisation may be applied to the detection of many different targets, simply by modifying the binder sequence in the linker.

In some embodiments, the linker nucleotide sequence is comprised within a nucleic acid having a stem-loop secondary structure and all or part of the binder sequence is comprised within a loop of the stem-loop structure and all or part of the trigger sequence is comprised within a stem of the stem-loop structure.

A "stem-loop secondary structure" as referred to herein may comprise one or more double stranded stem portions and one or more regions of unpaired nucleotide residues forming a loop portion.

In some embodiments, the trigger portion is generally of a length sufficient to enable denaturation or dissociation of a stem portion when the linker binds to the target. That is, hybridisation of the binder generally effects denaturation or dissociation of a stem portion of the linker, thus exposing trigger nucleotide sequence. In light of the above, a stem-portion of the stem loop structure generally comprises at least 5 nucleotides and may extend to about 50 nucleotides in length. However, the present invention should not be considered limited to any specific length of sequence and other sequence lengths may be used that provide the functionality described above.

In some embodiments, the linker is designed such that the melting temperature of the binder, when hybridised to the target, is higher than the melting temperature of the stem of the linker. In some embodiments, the melting temperature of the binder sequence when hybridised to the target is about 1°C, about 2°C, about 3°C, about 4°C, about 5°C, about 6°C, about 7°C, about 8°C, about 9°C, about 10°C, about 11°C, about 12°C, about 13°C, about 14°C, about 15°C, about 16°C, about 17°C, about 18°C, about 19°C or about 20°C higher than the melting temperature of the stem of the linker. In some embodiments, the melting temperature of the binder sequence when hybridised to the target is about 7°C higher than the melting temperature of the stem of the linker.

In some embodiments when the target is present in the sample, a heat or chemically denatured linker binds to the target (via the binder sequence) in preference to reverting to its native stem/loop structure following heat or chemical denaturation followed by the introduction of renaturation conditions.

The stem-portion of the stem-loop structure may be completely homologous, i.e. no mismatches in the hybridising sequences. Alternatively, the stem portion may include one or more mis-matched base pairs. For example, mis-matches may be useful in promoting denaturation of the stem portion of the stem loop structure during the method of the present invention and/or to allow hybridisation between the tether and the trigger sequence in the stem portion. Features such as mismatches and/or overhangs may also be incorporated into the stem to provide an appropriate melting temperature difference to be obtained without the requirement for an excessively long target-complementary sequence.

In some embodiments, the binder sequence may not be wholly within a loop, and may also be part of the stem. Similarly, the trigger sequence may not necessarily be wholly contained within a stem portion, and may extend into a loop portion.

In some embodiments, the binder and trigger portions of the linker may be separated by one or more nucleotide residues, partially overlapping, completely overlapping or one region may be contained within the other.

Typically, the linker nucleotide sequence will be of a type of nucleic acid which is not digestible by the reporter enzyme or the primary nuclease. Moreover, at least the trigger of the linker nucleotide sequence should be a suitable nucleic acid to form a substrate for the primary nuclease when the trigger is hybridized to the tether nucleotide sequence. In some embodiments, at least the trigger in the linker nucleotide sequence comprises DNA.

As set out above, the method of the present invention also contemplates the use of a reporter enzyme tethered to a solid support via a tether nucleotide sequence.

Reference herein to a "solid support" refers to any solid support to which a reporter enzyme may be attached via a tether nucleotide sequence. Examples of suitable solid supports include, for example, microscopy slides, chips, beads, dipsticks, nanotubes and the like. The solid support may be composed of any suitable material, including, for example, hydrophobic polymers or plastics, hydrophilic polymers or plastics, glass, cellulose-based materials and the like.

Such solid supports may also have surface modifications to improve attachment of a tether nucleotide sequence to the surface. For example, surface modifications of solid supports to facilitate nucleic acid attachment are described by Oh et al. (OMICS: A Journal of Integrative Biology 10(3): 327-343, 2006).

In some embodiments, a ligand may be attached to the solid support and a binding partner for the ligand may be incorporated into the tether nucleotide sequence, thus facilitating attachment of the tether nucleotide sequence to the solid support. For example, in some embodiments, avidin or streptavidin may be bound to the solid support, while biotin is incorporated into the tether nucleotide sequence. In this way binding between the avidin or streptavidin (on the solid support) and the biotin (in the tether nucleotide sequence) facilitates immobilization of the tether nucleotide sequence on the solid support. Other suitable ligand partners would be evident to people skilled in the art.

The tether nucleotide sequence must include at least a portion to which the trigger nucleotide sequence in the linker can hybridise under the conditions of the assay.

The tether nucleotide sequence may be any suitable species of nucleic acid with the proviso that the species of nucleic acid comprises a species of nucleic acid which is not digestible by the reporter enzyme. Furthermore, the species of nucleic acid must also be a species of nucleic acid that can be digested by the primary nuclease when a trigger sequence is hybridised to the tether nucleotide sequence. Based on the above design considerations, a person skilled in the art would readily ascertain a suitable species of nucleic acid for use as a tether nucleotide sequence.

In some embodiments, the tether nucleotide sequence comprises single stranded DNA. In these embodiments, a suitable primary nuclease would include a restriction endonuclease which can cleave double stranded DNA formed by the hybridisation of a DNA trigger nucleotide sequence to the DNA tether nucleotide sequence.

For example, in some embodiments, the tether nucleotide sequence comprises the nucleotide sequence set forth in SEQ ID NO: 1. In some embodiments wherein the tether nucleotide sequence comprises SEQ ID NO: 1, the trigger nucleotide sequence may comprise the nucleotide sequence set forth in SEQ ID NO: 2. Hybridisation between SEQ ID NO: 1 and SEQ ID NO: 2 may form a recognition/cleavage site for the restriction endonuclease *Aci*I and, therefore, *Aci*I may be used as a primary nuclease in embodiments of the invention which utilise SEQ ID NOs: 1 and 2 as the tether nucleotide sequence and trigger nucleotide sequence, respectively.

In some embodiments, the tether nucleotide sequence comprises single stranded RNA. In these embodiments, a suitable primary nuclease would include an RNase H which can cleave the RNA portion, i.e. the tether nucleotide sequence, in a DNA:RNA hybrid formed by the hybridisation of a DNA trigger nucleotide sequence to the RNA tether nucleotide sequence.

As would be appreciated, the tether nucleotide sequence may also comprise an RNA/DNA chimera wherein at least a portion of the tether nucleotide sequence may be RNA or DNA which hybridizes with the trigger to form a substrate for the primary nuclease (as described above), but which may also include other RNA or DNA portions which do not hybridise with the trigger to form a substrate for the primary nuclease.

As set out above, the method of the present invention also contemplates the use of a primary nuclease which specifically digests at least the tether nucleotide sequence in the hybrid formed between the trigger and the tether nucleotide sequence.

As referred to herein, a "nuclease" should be understood as any enzyme that can cleave the sugar-phosphate backbone of a nucleic acid. As such, the term "nuclease" should be understood to encompass both endonucleases and exonucleases. Furthermore, the nucleases contemplated for use in accordance with the present invention may be deoxyribonucleases (which cleave DNA) or ribonucleases (which cleave RNA). Nucleases that may be used in accordance with the present invention include, for example, restriction endonucleases, nucleases that cleave at sequence mis-matches, S1 nuclease, T7 endonuclease I, T4 endonuclease VII, CEL I (a plant-specific extracellular glycoprotein that belongs to the S1 nuclease family), and ribonucleases such as RNase A or RNase H.

The primary nuclease may be any suitable nuclease which digests the hybrid formed between the trigger and the tether nucleotide sequence. Typically, the primary nuclease should be chosen so that it cannot digest any of the other nucleic acid components of the assay as described herein. The choice of suitable primary nuclease will be evident to people skilled in the art based on the nature of the trigger and tether nucleotide sequences, as described herein.

In some embodiments, the primary nuclease comprises a restriction endonuclease.

As referred to herein, a "restriction endonuclease" refers to any endonuclease that binds to double-stranded DNA at a specific nucleotide sequence and then, if both strands of the DNA lack appropriate modification at that sequence, cleaves the DNA either at the recognition sequence or at another site in the DNA duplex. A wide array of restriction endonucleases with different recognition sites and different cleavage sites would be readily ascertained by people skilled in the art. For example, a range of restriction endonucleases may be sourced from New England Biolabs (Ipswich, MA, USA).

Typically, the primary nuclease may be a restriction endonuclease when both the tether and trigger nucleotide sequences hybridise to form a double stranded DNA which includes a recognition/cleavage site for the restriction endonuclease.

Reference herein to a "recognition/cleavage site" for a restriction endonuclease should be understood to include a nucleotide sequence which can be recognised by and cleaved by a particular restriction endonuclease.

In some embodiments, the primary nuclease comprises an RNase H.

RNase H (EC 3.1.26.4) is a ribonuclease that cleaves the 3'-O-P-bond of RNA in a DNA/RNA duplex to produce 3'-hydroxyl and 5'-phosphate terminated products. RNase H is a non-specific endonuclease and catalyzes the cleavage of RNA via a hydrolytic mechanism, aided by an enzyme-bound divalent metal ion.

In some embodiments, the primary nuclease may be an RNase H wherein the tether comprises at least a portion of RNA and the trigger nucleotide sequence comprises DNA and these molecules hybridise to form an RNA/DNA duplex. In this way, RNase H may selectively digest the RNA tether nucleotide sequence in a hybrid formed between an RNA tether nucleotide sequence and a DNA trigger nucleotide sequence.

As set out above, the method of the present invention also contemplates the use of a reporter enzyme. The "reporter enzyme" contemplated includes any enzyme for which an activity may be detected. For example, the reporter enzyme may be an enzyme which digests a substrate to produce a detectable product; the reporter enzyme may synthesise a detectable product from one or more substrates; or the reporter enzyme may act on a substrate to directly generate a signal such as luminescence, fluorescence, colour or temperature change (e.g. heat). A range of suitable enzymes would be readily ascertained by those of skill in the art. However, in general, enzymes which exhibit high specific activity and thus generate a high amount of product or signal per unit time may be generally desirable from a signal amplification perspective in the method of the present invention.

Examples of suitable reporter enzymes may include: nucleases, proteases, thrombin, enzymes which act on a chromogenic substrate or a fluorescent protein (for example, peroxidases such as HRP, glucorinidases such as GUS and galactosidases such as beta-galactosidases), transferases, phosphatases, oxidoreductases, synthases, hydrolases, lyases, isomerases and ligases.

In some embodiments, the reporter enzyme comprises a nuclease. Typically any nuclease used as a reporter enzyme should not be able to digest either the tether nucleotide sequence or the linker nucleic acid.

In some embodiments, the reporter enzyme comprises an RNase. In some embodiments, the RNase is RNase A.

Reference herein to an "RNase A" should be understood as any endonuclease which cleaves single stranded RNA. Examples of RNase A are described under EC 3.1.27.5

In these embodiments, the activity of the reporter enzyme would typically be detected by detecting digestion of an RNA substrate. Furthermore in these embodiments, neither the linker nucleic acid nor the tether nucleotide sequence would comprise unmodified single stranded RNA (which is digestible by RNase A).

In some embodiments, the reporter enzyme comprises a restriction endonuclease. In these embodiments, the activity of the reporter enzyme would typically be detected by detecting digestion of a double stranded DNA molecule wherein the double stranded DNA molecule comprises a recognition/cleavage site for the restriction endonuclease. In these embodiments neither the linker nucleic acid nor the tether nucleotide sequence should comprise a double stranded DNA recognition/cleavage site for the restriction endonuclease.

In some embodiments, the reporter enzyme comprises thrombin. In these embodiments, the detection of the reporter enzyme would typically be detected by proteolytic digestion of a peptide substrate for thrombin. Typically, thrombin cleaves between the Arg and Gly residues in the polypeptide sequence: Leu-Val-Pro-Arg-Gly-Ser.

As set out above, the method of the present invention contemplates detecting the activity of the reporter enzyme released from the solid support wherein the activity of the reporter enzyme released from the solid support is indicative of the presence of the target in the sample.

In some embodiments, the activity of the reporter enzyme is detected by determining the activity of the released reporter enzyme on a substrate for the reporter enzyme.

In some embodiments, the activity of the reporter enzyme released from the solid support is detected by determining the activity of the released reporter enzyme on a substrate which is temporally separated from the reporter enzyme tethered to the solid support.

Reference herein to a substrate which is "temporally separated" from the reporter enzyme tethered to the solid support refers to any separation in time between the substrate and the reporter enzyme tethered to the solid support. Typically, this may involve removal of reporter enzyme tethered to the solid support from an assay prior to the addition of the substrate to the assay. In this way, reporter enzyme released from a solid support will remain in the assay when the substrate is added, while reporter enzyme still tethered to the solid support will be removed from the assay.

In some embodiments, removal of reporter enzyme tethered to the solid support may involve separation of the solid support from the assay. For example, magnetic bead based solid supports may be removed from an assay under the influence of a magnetic field, other bead based solid supports may be removed from an assay by size based selection (e.g. filtration and/or centrifugation), dipstick based solid supports may be withdrawn from an assay, well or tube based solid supports may be removed from an assay by aspiration of the assay fluid from the well or vessel, and the like.

Alternatively, and depending on the nature of the solid support, reporter enzyme released from the solid support may be removed from the assay, for example by aspiration away from reporter enzyme still tethered to the solid support. The released reported enzyme may then be added to the substrate to complete the assay.

In some embodiments, the substrate which is temporally separated from the reporter enzyme tethered to the solid support is itself immobilised to a solid support.

In some embodiments, the activity of the reporter enzyme released from the solid support is detected by determining the activity of the released reporter enzyme on a substrate which is spatially separated from the reporter enzyme tethered to the solid support.

Reference herein to a substrate which is "spatially separated" from the reporter enzyme tethered to the solid support refers to any separation in space between the substrate and the reporter enzyme tethered to the solid support. Typically, the spatial separation includes a distance sufficient to prevent reporter enzymes tethered to the solid support from acting on the spatially separated substrate. Furthermore, the spatial separation is typically such that reporter enzyme released from a solid support can traverse the spatial separation and act on the substrate. In this way, only reporter enzyme released from the solid substrate can act on the spatially separated substrate.

In some embodiments, spatial separation of the substrate involves immobilisation of the substrate to a solid support which is spatially separated from the solid support comprising the tethered reporter enzyme. The solid support comprising the substrate may be a spatially separated region of the solid support comprising the tethered reporter enzyme or may be a separate solid support. In any event, reporter enzyme released from the solid support can traverse the spatial separation (e.g. by diffusion through a fluid) and act on the spatially separated substrate.

Immobilisation of a substrate to a solid support may be done using any suitable means based on the nature of the substrate and the solid support. Such immobilisation methods would be readily ascertained by a person skilled in the art.

The nature of the substrate used in the assay will depend on the nature of the reporter enzyme. For example, at least a portion of the substrate must be a substrate for the reporter enzyme released from the solid support. However, other portions of the substrate may not be a substrate for the reporter enzyme, and in some cases, this would be desirable (for example, wherein a feedback trigger is to be incorporated into the substrate as described in detail below).

Furthermore, the substrate used in the assay should not be digestible by the primary nuclease.

Examples of suitable combinations of reporter enzyme and substrate are provided below:
RNase A digesting a single stranded RNA substrate;
a restriction endonuclease digesting a DNA substrate;
RNase H digesting the RNA portion of an RNA/DNA duplex substrate;
a protease (e.g. thrombin) digesting a peptide substrate;
an enzyme acting on a chromogenic substrate to generate an optically detectable product (e.g. peroxidases such as HRP, glucorinidases such as GUS and galactosidases such as beta-galactosidases);
a biosynthetic enzyme acting on a substrate to generate a product which is detectably distinct (e.g. by molecular mass, pl or addition of a detectable label) from the initial substrate;
and the like.

Where the reporter enzyme is a nuclease or protease which digests a nucleic acid or peptide substrate, respectively, digestion of the substrate may be detected by any suitable means. Reference herein to "digestion" of a substrate should be understood to include complete digestion of the substrate into nucleotide or amino acid monomers or partial digestion of the substrate into any product of lower molecular weight than the initial substrate.

For example, digestion of a nucleic acid or peptide substrate into a lower molecular weight product may be determined by electrophoretic methods, staining methods, the release of a labelled nucleotide or peptide from an immobilised substrate, cleavage of a fluorophore/quencher labelled nucleic acid or peptide to release a fluorophore from quenching, and the like.

In some embodiments, a fluorophore is bound to the substrate and a quencher, which quenches the fluorescence of the fluorophore, is also bound to the substrate, wherein digestion of all or part of the substrate reduces or eliminates the quenching of the fluorophore by the quencher. Exemplary fluorophores and quenchers would be readily ascertained by people skilled in the art. In this regard, reference is made to the review of Marras (Methods Mol. Biol. 335, 3-16, 2006). In the above embodiments, digestion of the substrate allows dissociation of the fluorophore and quencher and thus allows the generation of a signal by the fluorophore.

In some embodiments, the method further comprises providing an intermediary molecule which is spatially separated from the reporter enzyme tethered to the solid support, wherein the intermediary molecule may be digested by reporter enzyme released from the solid support and wherein digestion of the intermediary molecule releases a feedback trigger which can hybridise to the tether nucleotide sequence to form a hybrid which may be digested by the primary nuclease.

In some embodiments, the feedback trigger may be incorporated into the substrate for the reporter enzyme and thus, in these embodiments, a single molecule performs the functions of both the substrate for the reporter enzyme and the intermediary molecule. Alternatively, in some embodiments, the substrate for the reporter enzyme and the intermediary molecule may be separate molecules.

In some embodiments, the intermediary molecule is immobilised to a solid support. This may be the case when the intermediary molecule and the substrate for the reporter enzyme are the same molecule or separate molecules.

In some embodiments, the feedback trigger in the intermediary molecule comprises a nucleotide sequence that is not digestible by the reporter enzyme. Typically, however, the feedback trigger comprises a nucleotide sequence which can hybridise with the tether nucleotide sequence to form a substrate for the primary nuclease. It is generally desirable that the feedback trigger is not digestible by the reporter enzyme to prevent digestion and therefore destruction of the feedback trigger by the reporter enzyme. However, in some embodiments, particularly those wherein the feedback trigger is incorporated into the substrate for the reporter enzyme, a portion of the molecule containing the feedback trigger may be a substrate for the reporter enzyme.

For example, in some embodiments, the intermediary molecule may comprise a nucleic acid molecule wherein part of the nucleic acid molecule may be digested by a reporter nuclease, to release a feedback trigger nucleic acid which is not digestible by the reporter nuclease. For example, the intermediary molecule may be an RNA/DNA hybrid wherein the RNA portion of the intermediary is digestible by an RNase (such as RNase A) to release a DNA feedback trigger which can hybridise with an RNA tether to form a. substrate for a primary nuclease being RNase H, or hybridise with a DNA tether nucleotide sequence to form a substrate for a primary nuclease being a restriction endonuclease.

In some embodiments, the intermediary molecule may comprise a nucleic acid-peptide conjugate wherein the peptide portion of the intermediary may represent a substrate for a protease reporter enzyme to release a nucleic acid (DNA or RNA) feedback trigger which can hybridise with an RNA or DNA tether nucleotide sequence to form a substrate for a primary nuclease as hereinbefore described.

In some embodiments, detecting the activity of the reporter enzyme released from the solid support comprises detecting digestion of the intermediary molecule. Thus, in these embodiments, the functions of the intermediary molecule and the substrate for the reporter enzyme are again incorporated into the one molecule.

Further disclosed is a solid support comprising a reporter enzyme tethered to the solid support via a tether nucleotide sequence.

In some embodiments, the reporter enzyme may be any reporter enzyme as hereinbefore described with reference to the first aspect of the invention.

In some embodiments, the tether nucleotide sequence may be any tether nucleotide sequence as hereinbefore described with reference to the first aspect of the invention. In some embodiments, the tether nucleotide sequence comprises single stranded DNA which is not digestible by the reporter enzyme. In some embodiments the tether nucleotide sequence comprises single stranded RNA which is not digestible by the reporter enzyme.

In some embodiments, the solid support further comprises a substrate for the reporter enzyme, wherein the substrate is spatially separated from the reporter enzyme tethered to the solid support, as hereinbefore described.

In some embodiments, the solid support further comprises an intermediary molecule which is spatially separated from the reporter enzyme tethered to the solid support.

As set out above, in some embodiments, a feedback trigger may be incorporated into a substrate for the reporter enzyme and thus, in these embodiments, a single molecule performs the functions of both the substrate for the reporter enzyme and the intermediary molecule. Alternatively, in some embodiments, the substrate for the reporter enzyme and the intermediary molecule may be separate molecules.

Finally, reference is made to standard textbooks of molecular biology that contain methods for carrying out basic techniques encompassed by the present invention, including DNA restriction, ligation and/or conjugation for the generation of the various genetic constructs described herein. See, for example, Maniatis et al., Molecular Cloning: A Laboratory Manual (Cold Spring Harbor Laboratory Press, New York, 1982) and Sambrook *et al.* (2000, *supra*).

Reference will now be made to the following non-limiting experimental examples which embody the above general principles of the invention. However, it is to be understood that the following description is for the purpose of describing particular embodiments only and is not intended to be limiting with respect to the above description.

### EXAMPLE 1

### Target Mediated Release of Tethered Enzyme

As shown in Figure 1, some embodiments of the present invention described herein are based on target-mediated release of a tethered reporter enzyme (100) from a solid support (110). The presence of a target nucleotide sequence (120) converts a portion of the tether nucleotide sequence (130) into a specific substrate (140) of a primary nuclease (150) present in the assay. Following formation of the primary nuclease's substrate (140), the tether (130) is immediately cleaved, consequently separating reporter enzyme (100) from the solid support (110). Free reporter enzyme (105) is able to produce a detectable signal by acting on its particular substrate, which remains spatially or temporally separated from tethered reporter enzyme (100) molecules.

For low copy number targets, the system can be modified such that for each target copy present, multiple reporter enzyme (100) molecules are released from their solid support (110), thus enhancing signal amplification.

Specific target detection is enabled through hybridisation of a binder nucleotide sequence (171) present in the loop portion (173) of the linker nucleotide sequence (170) to the target nucleic acid (120) following denaturation and renaturation. Once bound to the target (120), the linker nucleotide sequence (170) molecule is unable to re-fold into its native secondary structure. Non-target-bound linkers will however refold under renaturing conditions.

Dissociation of the complementary strands in the stem portion (174) of the linker (170) due to target (120) binding allows a trigger nucleotide sequence (172) to bind to the tether nucleotide sequence (130) attaching reporter enzyme (100) to the solid support. Once bound, the trigger (172) and tether (130) form the required substrate (140) for primary nuclease (150).

Importantly, the stem portion (174) of the linker molecule (170) is designed such that it is protected from digestion or cleavage by reporter enzyme (100) or (105), primary nuclease (150) or any other enzymes used in the assay.

The design of the linker (170) and tether (130) is dependent on the choice of reporter enzyme (100) and primary nuclease (150) for the method. For example, in some embodiments, primary nuclease (150) may be a restriction endonuclease and reporter enzyme (100) RNase A. In these embodiments, when the trigger (176) is bound to the tether (130), the substrate (140) comprises double stranded DNA which may be digested by a restriction endonuclease to cleave the tether (130). In these embodiments, the stem portion of the linker (174) is devoid of the restriction endonuclease's recognition site through the use of a mismatch or recessed terminus. Similarly, in these embodiments the linker (170) also contains no unmodified ribonucleotides so as not to be a substrate for RNase A.

In a second example, the primary nuclease (150) may be RNase H and reporter enzyme (100) a restriction endonuclease. In these embodiments, hybridisation between a DNA trigger sequence (172) and an RNA-based tether (130) forms a substrate (140) comprising a DNA:RNA hybrid, the RNA portion of which may be digested by RNase H thus cleaving tether (130). In these embodiments, providing each strand of the stem portion (174) is DNA lacking the restriction enzyme's recognition sequence; the linker will remain intact throughout the assay.

Following the separation of reporter enzyme (100) from the solid support (110), a signal is generated by means of the enzyme (105) acting on its previously spatially- or temporally-separated substrate. For example, if reporter enzyme (100/105) is RNase A, a signaling substrate (160) may comprise of dual-labeled RNA oligonucleotide tethered to a second solid support (115). The second solid support may be a distinct solid support or may be a region of solid support (110) which is sufficiently spatially separated from the tethered reporter enzymes (100) to prevent the tethered reporter enzymes (100) from digesting signaling molecule (160). Once in close proximity, released RNase A reporter enzymes (105) can rapidly cleave the signaling substrate (160), resulting in a detectable signal via the separation of a fluorophore (162) and quencher (164). As will be appreciated by a person skilled in the art, the fluorophore (162) and quencher (164) may also be reversed from the positions shown in Figure 1.

When oriented as shown in Figure 1, fluorescence would be read in solution, while if reversed, fluorescence would be read at the surface of the support. Significant signal amplification occurs due to the highly processive nature of RNase A.

### EXAMPLE 2

### Temporal Separation of Tethered Enzyme and Substrate

As illustrated in Figure 2, in some embodiments, enzyme (100) molecules may be tethered to magnetic beads (210) via a tether nucleotide sequence (130). As previously described, the presence of the target nucleic acid (120) sequence allows a reporter enzyme (100) molecule to be separated from its magnetic bead (210) through the action of the linker (170) and nuclease (150) on its tether nucleotide sequence (130).

Following magnetic removal of the beads (210) and tethered reporter enzyme (100) molecules, remaining free reporter enzyme (105) molecules are able to generate a signal upon the addition of an appropriate signaling substrate.

In such a system, the separation of the tethered reporter enzyme (100) molecules and signaling molecules may be considered temporal rather than spatial. Many combinations and variations of this system are possible depending on the identity of reporter enzyme (100) and primary nuclease (150). For example, the reporter enzyme (100) may be a restriction endonuclease and the signaling molecule may be a molecular break-light (260), as shown in Panel B of Figure 2 or enzyme (100) may be thrombin and may initiate a blood coagulation cascade as shown in Panel A of Figure 2.

### EXAMPLE 3

### Trigger Nucleotide Sequence Recycling

Augmentation of the basic system can lead to additional signal amplification through the release of multiple tethered reporter enzymes (100) for each target copy present. With reference to Figures 1 and 2, during cleavage of the tether (130) attaching reporter enzyme (100) to a solid support (110/210); the trigger sequence (172) of the linker (170) may remain intact after cleavage of a tether (130) and subsequently bind to another tether (130), which is then cleaved, and so on. In an example of such a system the primary nuclease (150) may be RNase H. RNase H, as mentioned previously, only digests the RNA portion of an RNA:DNA hybrid and leaves the DNA portion intact. Thus, if the tether (130) is RNA and at least the trigger sequence (172) in the linker (170) is DNA, only the tether (130) is cleaved in the DNA:RNA hybrid substrate (140) by nuclease (150) and the trigger sequence (172) is free to bind to another tether (130).

### EXAMPLE 4

### Feedback Loop

Additional amplification through the release of multiple reporter enzyme (100) molecules for each target nucleic acid (120) present can also be achieved through the induction of a feedback loop. Such a system involves intermediary molecules (300) bound to a solid support (320) which is spatially-separated from tethered reporter enzyme (100) molecules. The intermediary molecules (300) may be designed such that they include a feedback trigger nucleotide sequence (310) which is complementary to a portion of the tether (130) of reporter enzyme (100). In some embodiments, the feedback trigger sequence (302) may share some sequence identity to the trigger sequence (172) in the linker (170). A second portion of the intermediary nucleotide sequence (300), proximal to the solid support (310), is the substrate (304) for the enzyme (105). For example, if the enzyme (105) is RNase A, the feedback trigger sequence (302) in the intermediary nucleotide sequence (300) may comprise DNA, which is not digested by RNase A, while the substrate sequence (304) may comprise a sequence of ribonucleotides. Accordingly, free RNase A (105), released via the presence of the target nucleic acid (120), can digest the RNA portion (304) of the intermediary sequence (300) and allow the DNA feedback trigger (302) to separate from its solid support (310) and be free in solution. The DNA feedback trigger (302) can then bind to a tether (130). This binding forms a substrate (340) for the nuclease (150) to release an additional molecule of RNase A (100) which in turn can release a feedback trigger (302). The recurrent release of RNase A (100) and feedback triggers (302) from their spatially-separated solid supports constitutes a feedback loop.

A signaling function can also be incorporated into the intermediary molecule (300) by the addition of a fluorophore (306) and quencher (308). As will be appreciated by a person skilled in the art, the fluorophore (306) and quencher (308) may also be reversed from the positions shown in Figure 3. When oriented as shown in Figure 3, fluorescence would be read in solution, while if reversed, fluorescence would be read at the surface of the support. Alternatively, a separate signaling molecule may be used (not shown). As all RNase A (100) may be released from their solid support (110), independent of the number of target copies in solution, the potential detection sensitivity of the system is extremely high.

### EXAMPLE 5

### Tethered Enzyme Mediated Nucleic Acid Detection

Initially, a restriction endonuclease was selected as the primary nuclease and optimal sequences for the tether and trigger determined. For highly specific cleavage of double-stranded DNA containing its recognition motif, the restriction endonuclease *Aci*I was chosen.

A single-stranded DNA tether of 61 nucleotides was designed and synthesized:
DUAL BIOTIN-

GGGTGTTGTTGGGTTTGTGGTGGATGCCGCCTCAGCGACTCGGGTGTGTTG
TGTGTGGTGT-AMINE (SEQ ID NO: 1)

The non-trigger binding portion of the tether was comprised solely of thiamine and guanine bases, with an aim of preventing the formation of complex secondary and tertiary structures under reaction conditions. If sufficiently stable, such structures could hinder access of the trigger and primary nuclease and reduce the sensitivity of the biosensor.

The trigger (CCGAGTCGCTGAGGCGGCATCCAC; SEQ ID NO: 2) was designed to be of sufficient length to ensure a strong and stable bond to the tether. The tether/trigger duplex is shown in Figure 4.

Initially, polyacrylamide gel-based assays were performed to ensure that the trigger could bind to the tether and, subsequently, *Aci*I could cleave the trigger/tether duplex, particularly when the tether was bound to a streptavidin-coated magnetic bead (Figure 5).

Briefly, one µmol of the tether oligonucleotide (from a 100 µM stock solution) was bound to 750 µg of washed M-280 streptavidin-coated beads (Invitrogen) in 150 µl of binding buffer (5 mM Tris-HCl, pH7.5; 0.5 mM EDTA and 1 M NaCl), as per the manufacturer's instructions. Three washes with 400 µl binding buffer were performed to remove unbound tether, then the beads re-suspended in 35 µl NEBuffer 3 (100 mM NaCl; 50 mM Tris-HCl; 10 mM MgCl₂; 1 mM Dithiothreitol; pH 7.9 @ 25°C), a buffer in
which *Aci*I Is highly active. 10 µl of the re-suspended beads were then aliquoted into new 1.5 ml tubes for each of the three treatments: +trigger +*Aci*I; -trigger +*Aci*I; and trigger only (+trigger -*Aci*I). 15 pmoles (100 µM stock) trigger was added to the +trigger treatments and 1 µl *Aci*I (20 u/µl) was added to the +*Aci*I treatments. Reactions were supplemented with 20 µg Bovine Serum Albumin (BSA) and made up to 20 µl with dH₂O. Each reaction was agitated at 1200 rpm on a Thermomixer (Eppendorf) set at 37°C. Subsequently, the beads and bound nucleic acids were removed and discarded and the supernatant reserved for electrophoresis.

For the non-bead-bound controls, 15 pmoles of tether and 15 pmoles of trigger were added to a buffered solution (100 mM NaCl; 50 mM Tris-HCl; 10 mM MgCl₂; 1 mM Dithiothreitol; pH 7.9 @ 25°C) supplemented with 20 µg of BSA. -trigger and trigger only controls were also aliquoted. 1 µl *Aci*I (20 units/µl) was added to each reaction and the total volume made up to 20 µl with dH₂O. Each reaction was agitated at 1200 rpm in a Thermomixer set at 37°C.

All reactions, along with a low molecular weight ladder (NEB), were electrophoresed on a 10% polyacrylamide gel at 200 volts for 20 minutes then stained in ethidium bromide and visualised and photographed on a UV transilluminator.

Importantly, as shown in Figure 5, a DNA band of the correct size was evident for the treatment to which the trigger and *Aci*I had been added and absent in the no trigger control. This result indicated that the trigger had bound to the tether and the resulting duplex was specifically digested by *Aci*I, with the cleaved non-bead-bound portion remaining in solution after bead removal. The lack of a band in the magnetic bead-bound control treatment lanes indicated that in the absence of the trigger or the absence of *Aci*I, the tether remained intact and was removed with the magnetic beads. For the non-bead-bound controls, the presence of the trigger allowed *Aci*I to cleave the duplex, leading to a band of reduced size in comparison to the non-cleaved no trigger control.

Following the successful testing of the tether and trigger designs, RNase A was selected as the reporter enzyme. RNase A (Sigma-aldrich) was conjugated to the tether using an S-HyNic conjugation kit (SoluLink) as per the manufacturer's instructions.

To test the full biosensor assay, 1 mg of M-280 beads were washed twice in 200 µl dH₂O then re-suspended in 200 µl reaction buffer (100 mM NaCl; 50 mM Tris-HCl; 10 mM MgCl₂; 1 mM Dithiothreitol; pH 7.9 @ 25°C; 200 µg BSA). 25 µl of the RNase

A/tether conjugate reaction (conjugate - 10 µM) was added and allowed to bind for 15 minutes at room temperature followed by two washes in 140 µl of reaction buffer. The beads were split between two tubes (+ trigger and - trigger treatments) and buffer, trigger (if present) and *Aci*I were added. The final concentration of each reagent was 5 µM trigger if present; 25 units *Aci*I; 100 mM NaCl; 50 mM Tris-HCl; 10 mM MgCl₂; 1 mM Dithiothreitol; pH 7.9 @ 25°C. The reactions were agitated at 1200 rpm on a Thermomixer set at 37°C. Following removal of the magnetic beads, the remaining supernatant was diluted 100 times by volume with reaction buffer/BSA, then two 20 µl replicates for each treatment aliquoted into 100 µl PCR tubes (Axygen). Following addition of the signaling molecule (FAM-mArArUrGrGrCmA-Blk_FQ) to a final concentration of 50 nM, the reactions were analysed on a Rotorgene RG-3000 (Corbett) operating at a constant temperature of 37°C. In addition, a control comprising of signaling molecule (50 nM) in dH₂O was also analysed. Fluorescence readings were acquired on the FAM channel every 30 seconds. The results were graphed using Excel (Microsoft) and are shown in Figure 6.

The data indicate that when the trigger was present, a more intense signal was produced compared to when the trigger was absent. This signifies that the trigger had bound to the tether and subsequently the trigger/tether duplex had been cleaved by *Aci*I, hence releasing RNase A from the bead. Following removal of the beads, the remaining RNase A in solution was able to cleave multiple signaling molecules, thus generating a detectable signal. Some background signal was also present in the no trigger treatment, however, optimization to ensure all non-conjugated RNase A is removed prior to the biosensor reaction being carried out should address this background signal.

Also, it must be noted that, as used herein, the singular forms "a", "an" and "the" include plural aspects unless the context already dictates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

## Claims

1. A method for detecting a target nucleotide sequence in a sample, the method comprising:
providing a reporter enzyme tethered to a solid support via a tether nucleotide sequence;
providing a linker nucleotide sequence comprising a binder which can hybridise to the target nucleotide sequence, and a trigger which can hybridise to the tether nucleotide sequence only when the binder nucleotide sequence is bound to the target;
providing a primary nuclease which digests at least the tether nucleotide sequence in a hybrid formed between the trigger and the tether nucleotide sequence;
contacting a sample comprising the target nucleotide sequence with the reporter enzyme tethered to a solid support, the linker nucleotide sequence, and the nuclease, under suitable conditions wherein the binder hybridises to the target nucleotide sequence, the trigger then hybridises to the tether nucleotide sequence, the nuclease digests at least the tether nucleotide sequence in the hybrid formed between the trigger and the tether nucleotide sequence, and the reporter enzyme is released from the solid support; and
detecting the activity of the reporter enzyme released from the solid support wherein the activity of the reporter enzyme released from the solid support is indicative of the presence of the target in the sample.

2. The method of claim 1 wherein the linker nucleotide sequence is comprised within a nucleic acid having a stem-loop secondary structure and all or part of the binder is comprised within a loop of the stem-loop structure and all or part of the trigger is comprised within a stem of the stem-loop structure.

3. The method of claim 1 or 2 wherein at least the trigger in the linker nucleotide sequence comprises DNA.

4. The method of any one of claims 1 to 3 wherein the tether nucleotide sequence comprises single stranded DNA or single stranded RNA.

5. The method of claim 4 wherein the tether nucleotide sequence comprises single stranded DNA and the primary nuclease comprises a restriction endonuclease.

6. The method of claim 4 wherein the tether nucleotide sequence comprises single stranded RNA, wherein at least the trigger in the linker nucleotide sequence comprises DNA, and wherein the primary nuclease comprises an RNase H.

7. The method of any one of claims 1 to 6 wherein the reporter enzyme comprises a nuclease, a protease, thrombin, an enzyme which acts on a chromogenic substrate or a fluorescent protein.

8. The method of claim 7 wherein the reporter enzyme comprises an RNase, preferably RNase A, or comprises a restriction endonuclease.

9. The method of any one of claims 1 to 8 wherein the activity of the reporter enzyme released from the solid support is detected by determining the activity of the released reporter enzyme on a substrate which is temporally or spatially separated from the reporter enzyme tethered to the solid support.

10. The method of any one of claims 1 to 9 wherein the method further comprises providing an intermediary molecule which is spatially separated from the reporter enzyme tethered to the solid support, wherein the intermediary molecule may be digested by reporter enzyme released from the solid support and wherein digestion of the intermediary molecule releases a feedback trigger which can hybridise to the tether nucleotide sequence to form a hybrid which may be digested by the primary nuclease.

11. The method of claim 10 wherein the feedback trigger in the intermediary molecule comprises a nucleotide sequence that is not digestible by the reporter enzyme.

## Patentansprüche

1. Verfahren zum Nachweisen einer Ziel-Nukleotid-Sequenz in einer Probe, wobei das Verfahren folgendes umfasst:
Bereitstellen eines Reporterenzyms, das über eine Anbindungs-Nukleotid-Sequenz an einen festen Träger angebunden ist;
Bereitstellen einer Linker-Nukleotid-Sequenz, die einen Binder umfasst, der an die Ziel-Nukleotid-Sequenz hybridisieren kann, und einen Trigger, der nur dann an die Anbindungs-Nukleotid-Sequenz hybridisieren kann, wenn die Binder-Nukleotid-Sequenz an das Ziel angebunden ist;
Bereitstellen einer primären Nuklease, welche wenigstens die Anbindungs-Nukleotid-Sequenz in einer hybriden Form zwischen der Trigger- und der Anbindungs-Nukleotid-Sequenz verdaut;
Inkontaktbringen einer Probe, welche die Ziel-Nukleotid-Sequenz, mit dem an einen festen Träger angebundenen Reporterenzym, die Linker-Nukleotid-Sequenz und die Nuklease umfasst, unter geeigneten Bedingungen, wobei der Binder an die Ziel-Nukleotid-Sequenz hybridisiert, wobei der Trigger danach an die Anbindungs-Nukleotid-Sequenz hybridisiert, wobei die Nuklease wenigstens die Anbindungs-Nukleotid-Sequenz in dem zwischen dem Trigger und der Anbindungs-Nukleotid-Sequenz gebildeten Hybrid verdaut, und wobei das Reporterenzym von dem festen Träger freigesetzt wird; und
Nachweisen der Aktivität des von dem festen Träger freigesetzten Reporterenzyms, wobei die Aktivität des von dem festen Träger freigesetzten Reporterenzyms das Vorhandensein des Ziels in der Probe anzeigt.

2. Verfahren nach Anspruch 1, wobei die Linker-Nukleotid-Sequenz in einer Nukleinsäure vorhanden ist, die eine Haarnadelstruktur aufweist, und wobei der ganze oder ein Teil des Binders in einer Schleife der Haarnadelstruktur vorgesehen ist, und wobei der ganze oder ein Teil des Triggers in einem Stamm der Haarnadelstruktur vorgesehen ist.

3. Verfahren nach Anspruch 1 oder 2, wobei wenigstens der Trigger in der Linker-Nukleotid-Sequenz DNA umfasst.

4. Verfahren nach einem der Ansprüche 1 bis 3, wobei die Anbindungs-Nukleotid-Sequenz einsträngige DNA oder einsträngige RNA umfasst.

5. Verfahren nach Anspruch 4, wobei die Anbindungs-Nukleotid-Sequenz einsträngige DNA umfasst, und wobei die primäre Nuklease eine Restriktionsendonuklease umfasst.

6. Verfahren nach Anspruch 4, wobei die Anbindungs-Nukleotid-Sequenz einsträngige RNA umfasst, wobei wenigstens der Trigger in der Linker-Nukleotid-Sequenz DNA umfasst, und wobei die primäre Nuklease eine RNase H umfasst.

7. Verfahren nach einem der Ansprüche 1 bis 6, wobei das Reporterenzym eine Nuklease, eine Protease, Thrombin, ein Enzym, das als chromogenes Substrat oder als fluoreszierendes Protein wirkt.

8. Verfahren nach Anspruch 7, wobei das Reporterenzym eine RNase, vorzugsweise RNase A, umfasst oder eine Restriktionsendonuklease umfasst.

9. Verfahren nach einem der Ansprüche 1 bis 8, wobei die Aktivität des von dem festen Träger freigesetzten Reporterenzyms nachgewiesen wird durch Nachweis der Aktivität des freigesetzten Reporterenzyms auf einem Träger, der temporär oder räumlich getrennt ist von dem Reporterenzym, das an den festen Träger angebunden ist.

10. Verfahren nach einem der Ansprüche 1 bis 9, wobei das Verfahren ferner das Bereitstellen eines intermediären Moleküls umfasst, das räumlich getrennt ist von dem Reporterenzym, das an den festen Träger angebunden ist, wobei das intermediäre Molekül durch das von dem festen Träger freigesetzte Reporterenzym verdaut werden kann, und wobei die Verdauung des intermediären Moleküls einen Feedback-Trigger freisetzt, der an die Anbindungs-Nukleotid-Sequenz hybridisieren kann, um ein Hybrid zu bilden, das von der primären Nuklease verdaut werden kann.

11. Verfahren nach Anspruch 10, wobei der Feedback-Trigger in dem intermediären Molekül eine Nukleotid-Sequenz umfasst, die nicht von dem Reporterenzym verdaut werden kann.

## Revendications

1. Procédé permettant la détection d'une séquence de nucléotides cible dans un échantillon, le procédé comprenant les étapes consistant à :
fournir une enzyme rapporteur attachée à un support solide via une séquence de nucléotides d'attache ;
fournir une séquence de nucléotides de liaison comprenant un liant qui peut s'hybrider à la séquence de nucléotides cible, et un déclencheur qui peut s'hybrider à la séquence de nucléotides d'attache seulement lorsque la séquence de nucléotides de liaison est liée à la cible ;
fournir une nucléase primaire qui digère au moins la séquence de nucléotides d'attache dans un hybride formé entre le déclencheur et la séquence de nucléotides d'attache ;
mettre en contact un échantillon comprenant la séquence de nucléotides cible avec l'enzyme rapporteur attachée à un support solide, à la séquence de nucléotides de liaison, et à la nucléase, dans des conditions convenables, le liant s'hybridant à la séquence de nucléotides cible, le déclencheur s'hybridant alors à la séquence de nucléotides d'attache, la nucléase digérant au moins la séquence de nucléotides d'attache dans l'hybride formé entre le déclencheur et la séquence de nucléotides d'attache, et l'enzyme rapporteur étant libérée du support solide ; et
détecter l'activité de l'enzyme rapporteur libérée du support solide, l'activité de l'enzyme rapporteur libérée du support solide étant indicative de la présence de la cible dans l'échantillon.

2. Procédé selon la revendication 1, dans lequel la séquence de nucléotides de liaison est comprise au sein d'un acide nucléique possédant une structure secondaire tige-boucle et tout ou une partie du liant est compris au sein d'une boucle de la structure tige-boucle et tout ou une partie du déclencheur est compris au sein d'une tige de la structure tige-boucle.

3. Procédé selon la revendication 1 ou la revendication 2, dans lequel au moins le déclencheur dans la séquence de nucléotides de liaison comprend de l'ADN.

4. Procédé selon l'une quelconque des revendications 1 à 3, dans lequel la séquence de nucléotides d'attache comprend de l'ADN simple brin ou de l'ARN simple brin.

5. Procédé selon la revendication 4, dans lequel la séquence de nucléotides d'attache comprend de l'ADN simple brin et la nucléase primaire comprend une endonucléase de restriction.

6. Procédé selon la revendication 4, dans lequel la séquence de nucléotides d'attache comprend de l'ARN simple brin, dans lequel au moins le déclencheur dans la séquence de nucléotides de liaison comprend de l'ADN et dans lequel la nucléase primaire comprend une RNase H.

7. Procédé selon l'une quelconque des revendications 1 à 6, dans lequel l'enzyme rapporteur comprend une nucléase, une protéase, de la thrombine, une enzyme qui agit sur un substrat chromogène ou une protéine fluorescente.

8. Procédé selon la revendication 7, dans lequel l'enzyme rapporteur comprend une RNase, de préférence RNase A ou comprend une endonucléase de restriction.

9. Procédé selon l'une quelconque des revendications 1 à 8, dans lequel l'activité de l'enzyme rapporteur libérée du support solide est détectée en déterminant l'activité de l'enzyme rapporteur libérée sur un substrat qui est temporairement ou spatialement séparé de l'enzyme rapporteur attachée au support solide.

10. Procédé selon l'une quelconque des revendications 1 à 9, dans lequel le procédé consiste en outre à fournir une molécule intermédiaire qui est spatialement séparée de l'enzyme rapporteur attachée au support solide, dans lequel la molécule intermédiaire peut être digérée par l'enzyme rapporteur libérée du support solide et dans lequel la digestion de la molécule intermédiaire libère un déclencheur de rétroaction qui peut s'hybrider à la séquence de nucléotides d'attache pour former un hybride qui peut être digéré par la nucléase primaire.

11. Procédé selon la revendication 10, dans lequel le déclencheur de rétroaction dans la molécule intermédiaire comprend une séquence de nucléotides qui n'est pas digérable par l'enzyme rapporteur.
